(19) Europäisches Patentamt
European Patent Office
Office européen des brevets

(11) **EP 4 382 907 B1**

(12) **EUROPÄISCHE PATENTSCHRIFT**

(45) Veröffentlichungstag und Bekanntmachung des Hinweises auf die Patenterteilung:
**21.01.2026 Patentblatt 2026/04**

(21) Anmeldenummer: **23213024.5**

(22) Anmeldetag: **29.11.2023**

(51) Internationale Patentklassifikation (IPC):
***G01N 33/50*** *(2006.01)* ***G01N 33/574*** *(2006.01)*

(52) Gemeinsame Patentklassifikation (CPC):
**G01N 33/5011; G01N 33/57446;** G01N 2800/52

(54) **VERFAHREN ZUR PRÄDIKATION EINES PATIENTENINDIVIDUELLEN ANSPRECHENS AUF DAS FLOT-CHEMOTHERAPIE-SCHEMA**

METHOD FOR PREDICTING A PATIENT-SPECIFIC RESPONSE TO THE FLOT CHEMOTHERAPY SCHEME

PROCÉDÉ DE PRÉDICTION D'UNE RÉPONSE SPÉCIFIQUE À UN PATIENT À UN RÉGIME DE CHIMIOTHÉRAPIE À BASE DE FLOT

(84) Benannte Vertragsstaaten:
**AL AT BE BG CH CY CZ DK EE ES FI FR GB GR HR HU IE IS IT LI LT LU LV MC ME MK MT NL NO PL PT RO RS SE SI SK SM TR**

(30) Priorität: **09.12.2022 DE 102022132767**

(43) Veröffentlichungstag der Anmeldung:
**12.06.2024 Patentblatt 2024/24**

(73) Patentinhaber: **Technische Universität Dresden 01069 Dresden (DE)**

(72) Erfinder:
• **Prof. Dr. Stange, Daniel**
**01277 Dresden (DE)**
• **Dr. Schmäche, Tim**
**01307 Dresden (DE)**
• **Prof. Dr. Weitz, Jürgen**
**01069 Dresden (DE)**

(74) Vertreter: **Sperling, Thomas**
**Sperling, Fischer & Heyner**
**Patentanwälte**
**Tolkewitzer Straße 22**
**01277 Dresden (DE)**

(56) Entgegenhaltungen:
**EP-A1- 3 657 167    WO-A1-2022/144365**

• **ZOU JIALE ET AL: "Construction of gastric cancer patient-derived organoids and their utilization in a comparative study of clinically used paclitaxel nanoformulations", vol. 20, no. 1, 18 May 2022 (2022-05-18), XP093113716, ISSN: 1477-3155, Retrieved from the Internet <URL:https://link.springer.com/article/10.1186/s12951-022-01431-8/fulltext.html> [retrieved on 20240327], DOI: 10.1186/s12951-022-01431-8**
• **VOGL URSULA M. ET AL: "Ramucirumab plus paclitaxel or FOLFIRI in platinum-refractory advanced or metastatic gastric or gastroesophageal junction adenocarcinoma-experience at two centres", vol. 11, no. 2, 1 April 2020 (2020-04-01), pages 366 - 375, XP093146236, ISSN: 2078-6891, Retrieved from the Internet <URL:https://www.ncbi.nlm.nih.gov/pmc/articles/PMC7212104/pdf/jgo-11-02-366.pdf> [retrieved on 20240327], DOI: 10.21037/jgo.2020.03.10**
• **LORENZEN S ET AL: "Feasibility of perioperative chemotherapy with infusional 5-FU, leucovorin, and oxaliplatin with (FLOT) or without (FLO) docetaxel in elderly patients with locally advanced esophagogastric cancer", vol. 108, no. 3, 15 January 2013 (2013-01-15), London, pages 519 - 526, XP093113718, ISSN: 0007-0920, Retrieved from the Internet <URL:http://www.nature.com/articles/bjc2012588> [retrieved on 20240327], DOI: 10.1038/bjc.2012.588**

- **LI XIAODUN ET AL:** "Organoid cultures recapitulate esophageal adenocarcinoma heterogeneity providing a model for clonality studies and precision therapeutics", vol. 9, no. 1, 30 July 2018 (2018-07-30), UK, XP093146361, ISSN: 2041-1723, Retrieved from the Internet <URL:https://www.nature.com/articles/s41467-018-05190-9> [retrieved on 20240327], DOI: 10.1038/s41467-018-05190-9
- **LE COMPTE MAXIM ET AL:** "Patient-derived organoids as individual patient models for chemoradiation response prediction in gastrointestinal malignancies", CRITICAL REVIEWS IN ONCOLOGY/HEMATOLOGY, ELSEVIER, AMSTERDAM, NL, vol. 157, 28 November 2020 (2020-11-28), XP086454858, ISSN: 1040-8428, [retrieved on 20201128], DOI: 10.1016/J.CRITREVONC.2020.103190

- **SEIDLITZ THERESE ET AL:** "Human gastric cancer modelling using organoids", GUT MICROBIOTA, vol. 68, no. 2, 27 April 2018 (2018-04-27), UK, pages 207 - 217, XP093237226, ISSN: 0017-5749, DOI: 10.1136/gutjnl-2017-314549

# EP 4 382 907 B1

**Beschreibung**

Die Erfindung betrifft ein Verfahren zur Prädikation eines patientenindividuellen Ansprechens auf das FLOT-Chemotherapie-Schema, welches in der Klinik zur Behandlung des Ösophaguskarzinoms und des Magenkarzinoms eingesetzt wird.

Magenkarzinome und Speiseröhrenkarzinome zählen weltweit zu den am häufigsten auftretenden tödlichen Erkrankungen. Aufgrund des Fehlens früher klinischer Anzeichen ist Magenkrebs durch eine oft verzögerte Diagnose gekennzeichnet, was zu einer großen Anzahl von Patienten mit schlechter Prognose führt. Die Therapie umfasst chirurgische Eingriffe, bei welchen bösartiges Gewebe reseziert wird, und verschiedene Chemotherapien. Die Abkürzung FLOT bezeichnet ein Chemotherapie-Schema zur Behandlung von Magenkarzinomen und Adenokarzinomen des gastro-ösophagenalen Übergangs beziehungsweise des Ösophagus, welche als ösophago-gastrale Karzinome zusammengefasst werden können. Das FLOT-Chemotherapie-Schema besteht aus einer Kombination von vier Wirkstoffen. Diese sind 5-Fluorouracil (F), Folinsäure (Leucovorin) (L), Oxaliplatin (O) und Docetaxel (ein Chemotherapeutikum aus der Gruppe der Taxane) (T). Obwohl die FLOT-Chemotherapie im Vergleich mit anderen Chemotherapie-Schemata häufiger zu einem vollständigen Rückgang von Magenkarzinomen und ösophago-gastralen Karzinomen führt, ist das Ansprechverhalten und der damit einhergehende Therapieerfolg der FLOT-Chemotherapie bei betreffenden Patienten mit der Standard-Behandlung sehr unterschiedlich. So resultiert die Standard-Behandlung mit der FLOT-Chemotherapie nur bei etwa einem Drittel der Patienten in einer gewünschten Tumorregression und einhergehend einer verbesserten Überlebensrate. Bislang existieren keine verlässlichen Indikatoren, welche die Identifikation von gut oder schlecht auf eine FLOT-Chemotherapie ansprechende Patienten ermöglichen können. Patienten, bei denen eine FLOT-Chemotherapie nicht beziehungsweise nicht im gewünschten Maß anspricht, profitieren nicht relevant von der Therapie und leiden zudem unter den Chemotherapie-bedingten Nebenwirkungen, wie zum Beispiel Übelkeit, dem Hand-Fuß-Syndrom, Panzytopenie oder Fatigue. Diese Nebenwirkungen beeinflussen den Therapieverlauf nachteilig.

EP3657167 offenbart die Analyse der Therapieantwort unter Verwendung von Tumororganoiden von Patienten mit gastrointestinalem Krebs, wobei eine Kombination aus Fluorouracil oder Capecitabine in Kombination mit Irinotecan getestet wird.

Für den Therapieverlauf und für die Therapieaussichten von Patienten mit Magenkarzinom oder ösophago-gastralen Karzinom ist es daher von großer Bedeutung, wenn hinsichtlich der Anwendung des FLOT-Chemotherapie-Schemas vor Therapiebeginn eine Aussage über das Ansprechverhalten getroffen werden kann.

Die Aufgabe der Erfindung besteht somit darin, bei Patienten mit Magenkarzinom oder ösophago-gastralen Karzinom eine Vorhersage des Ansprechens auf eine FLOT-Chemotherapie treffen zu können, um die FLOT-Chemotherapie somit zur Eignung für Anwendungen in der personalisierten Medizin zu etablieren.

Die Aufgabe wird durch ein Verfahren mit den Merkmalen gemäß Patentanspruch 1 gelöst. Alternative oder zusätzliche Verfahrensschritte sind in den abhängigen Patentansprüchen angegeben.

Dem erfindungsgemäßen Verfahren liegt die Erkenntnis zugrunde, dass Dosis-Wirkungstests erfolgreich an unter physiologischen Bedingungen *in vitro* kultivierten Tumorzellen von ösophago-gastralen Karzinomen durchgeführt werden können, wobei die daraus gewonnenen Ergebnisse auf *in vivo* Anwendungen des in der klinischen Praxis angewendeten FLOT-Chemotherapie-Schemas übertragen werden können.

Bei dem erfindungsgemäßen Verfahren zur Prädikation eines patientenindividuellen Ansprechens auf ein FLOT-Chemotherapie-Schema bei ösophago-gastralen Karzinomen wird so vorgegangen, dass zunächst Zellen eines ösophago-gastralen Karzinoms, insbesondere eines Magenkarzinoms, eines Patienten bei physiologischen Bedingungen *in vitro* kultiviert werden. Das Verfahren sieht den Einsatz einer konkreten Testzusammensetzung zur Behandlung der kultivierten Patientenzellen vor. Diese Testzusammensetzung enthält mindestens eine Komponente ausgewählt aus einer Gruppe von Komponenten aufweisend 5-Fluorouracil, Oxaliplatin und Docetaxel. Die Konzentration der mindestens einen Komponente ist vorgegeben. Bei Testzusammensetzungen mit mehreren Komponenten können sich die Komponentenkonzentrationen unterscheiden. Die Testzusammensetzung kann somit eine oder mehrere der Komponenten, nämlich 5-Fluorouracil, Oxaliplatin oder Docetaxel, enthalten, wobei jede Komponente eine vorgegebene Komponentenkonzentration aufweist. Demzufolge kann die Testzusammensetzung verschiedene Kombinationen der Komponenten 5-Fluorouracil, Oxaliplatin, Docetaxel aufweisen. Die Testzusammensetzung als solche kann ebenfalls in unterschiedlichen Konzentrationen bereitgestellt werden.

Die kultivierten Patientenzellen werden mit mindestens einer vorgegebenen Konzentration der bereitgestellten Testzusammensetzung behandelt und inkubiert. Der Begriff Behandlung bezeichnet im Sinne des erfindungsgemäßen Verfahrens einen Vorgang, bei welchem die Patientenzellkultur mit der bereitgestellten Testzusammensetzung so in Kontakt gebracht wird, dass die in der Testzusammensetzung enthaltende(n) Komponente(n) auf die Zellen der Patientenkultur unter physiologischen Bedingungen einwirken kann/können. Nach einer vorgegebenen Inkubationsdauer wird eine Zellviabilität der Patientenzellen ermittelt, wobei anhand der Zellviabilität ein patientenindividuelles Ansprechen der Patientenzellen auf das FLOT-Chemotherapie-Schema abgeleitet wird.

Es hat sich überraschend gezeigt, dass eine zutreffende Aussage zum Ansprechverhalten auf das FLOT-Chemotherapie-Schema mittels einer Testzusammensetzung erzielt werden kann, welche lediglich eine der Komponenten 5-Fluorouracil, Oxaliplatin oder Docetaxel enthält. Es kann daher vorgesehen sein, dass die Testzusammensezung genau eine Komponente ausgewählt aus einer Gruppe von Komponenten aufweisend 5-Fluorouracil, Oxaliplatin und Docetaxel, enthält. In diesem Fall besteht die Testzusammensetzung aus der einen ausgewählten Komponente, welche in einem neutralen, nicht auf Zellen wirkenden Lösungsmittel enthalten ist. Vorteilhaft kann die Testzusammensetzung mit nur einer einzigen enthaltenen Komponente mit vergleichsweise geringerem Aufwand bereitgestellt werden.

Für die Bestimmung der Zellviabilität stehen unterschiedliche Verfahren zur Verfügung, welche nach verschiedenen Messprinzipien arbeiten. Die Verfahren umfassen mikroskopische, fluorimetrische, colorimetrische, luminometrische, durchflusscytometrische und Impedanzänderungsbasierte Methoden. Besonders einfach ist eine mikroskopische Auswertung von Zellveränderungen während der Inkubation oder die Erfassung von Änderungen im Stoffwechsel der Patientenzellen, beispielsweise durch Bestimmung eines Farbumschlages des Zellkulturmediums.

Die verfahrensgemäß ermittelte Zellviabilität der Patientenkultur liegt der Bewertung eines guten oder schlechten Ansprechens der Testzusammensetzung zugrunde, woraus letztlich ein Ansprechverhalten des betreffenden Patienten auf das in der klinischen Praxis eingesetzte FLOT-Chemotherapie-Schema abgeleitet wird. Ein gutes Ansprechen von Patientenzellen ist im Kontext einer Kohorte relativ zu Vergleichsproben zu beurteilen.

Die ermittelten Zellviabilitätswerte werden zur Bestimmung eines patientenspezifischen Wertes eingesetzt, anhand welchem im Vergleich mit einem vorgegebenen Schwellenwert eine Prädikation eines patientenindividuellen Ansprechens auf das FLOT-Chemotherapie-Schema abgeleitet wird.

Ein hier beschriebenes Verfahren kann in folgende Schritte zusammengefasst werden:

1. Bereitstellen von Zellen eines ösophago-gastralen Karzinoms, insbesondere eines Magenkarzinoms, eines Patienten,
2. *in vitro* Kultivierung der entnommenen Patientenzellen unter physiologischen Bedingungen,
3. Bereitstellen einer Testzusammensetzung, welche zumindest eine Komponente ausgewählt aus einer Gruppe von Komponenten aufweisend 5-Fluorouracil, Oxaliplatin und Docetaxel, enthält, wobei die Komponentenkonzentration in der Testzusammensetzung vorgegeben ist,
4. Behandeln der *in vitro* kultivierten Patientenzellen mit mindestens einer vorgegebenen Konzentration der Testzusammensetzung und Inkubieren für eine vorgegebene Inkubationsdauer,
5. Ermitteln der Zellviabilität der behandelten Patientenkultur und
6. Bewerten des Ansprechens der Patientenkultur auf die Testzusammensetzung anhand der ermittelten Zellviabilität und Ableiten einer Prädikation zum Ansprechen des Patienten auf das FLOT-Chemotherapie-Schema.

Das erfindungsgemäße Verfahren ist in Anspruch 1 aufgeführt.

Es kann vorgesehen werden, dass die Patientenkultur in Vertiefungen (*englisch wells*) von Zellkulturplatten geteilt wird und die einzelnen Vertiefungen mit den darin enthaltenen Patientenkulturen jeweils mit unterschiedlichen Konzentrationen der Testzusammensetzung behandelt werden. Dabei wird jede Vertiefung mit den darin enthaltenen Patientenzellen jeweils mit einer konkreten Konzentration der Testzusammensetzung behandelt. Auf diese Weise kann der Einfluss der Dosis der Testzusammensetzung auf die Patientenzellen berücksichtigt und in Form einer Dosis-Wirkungs-Kurve visualisiert werden.

Zur Auswertung der Zellviabilitätswerte ist vorgesehen, dass anhand der ermittelten Zellviabilitätswerte eines Patienten eine Dosis-Wirkungs-Kurve beziehungsweise eine Funktion der Dosis-Wirkung erstellt wird. Dabei wird die Dosis, welche der jeweils applizierten Konzentration der Testzusammensetzung entspricht, gegen die jeweils ermittelte Zellviabilität in Prozent aufgetragen. Der patientenspezifische Wert ($AUC_{rel}$) errechnet sich dann aus dem Quotient des Integrals der Dosis-Wirkungs-Kurve, beziehungsweise der Fläche unter der Kurve (*englisch area under the curve "AUC"*), und dem Integral der Funktion der Viabilitätswertmaxima ($AUC_{max}$) gemäß der Gleichung (1):

$$(1) \qquad\qquad AUC_{rel} = AUC/AUC_{max.}$$

Dabei entspricht der Wert $AUC_{max}$ dem Integral der maximalen, das heißt der unveränderten Viabilität. Ein patientenspezifischer Wert ($AUC_{rel}$) kleiner als ein vorgegebener Schwellenwert ($AUC_{threshold}$) bedeutet ein gutes Ansprechen und ein patientenspezifischer Wert größer als der Schwellenwert ($AUC_{threshold}$) bedeutet ein schlechtes Ansprechen des betreffenden Patienten auf das klinische FLOT-Chemotherapie-Schema. Es kann somit eine Vorhersage getroffen werden, ob das klinisch eingesetzte FLOT-Chemotherapie-Schema bei dem betreffenden Patienten einen Behandlungserfolg erzielen kann. Die Festlegung des Schwellenwertes ($AUC_{threshold}$) zur Beurteilung des Ansprechverhaltens kann auf empirisch ermittelten Daten beruhen. Beispielsweise kann das Ansprechen des Patienten an Hand von noch viablen Tumorzellen nach Chemotherapie des Patienten im Operationspräparat bestimmt werden. Sind beispielsweise weniger als 10 % der Tumorzellen noch viabel, wird von einem guten Ansprechen ausgegangen. Sind noch mehr als 10 % der Tumorzellen viabel, wird von einem schlechten Ansprechen ausgegangen. Entsprechende Ergebnisse können bei dem erfindungsgemäßen Verfahren zur Festlegung des Schwellenwertes ($AUC_{threshold}$) eingesetzt werden. Der Schwellenwert ($AUC_{threshold}$) wird auf Basis einer *in vivo* Dosis-Wirkung empirisch bestimmt. Es kann weiter vorgesehen werden, dass zur Bestimmung des Schwellenwertes ($AUC_{threshold}$) ein auf einem Computer ausgeführtes Programm angewendet wird, um anhand von *in vitro* ermittelten Dosis-Wirkungs-Werten einen Schwellenwert ($AUC_{threshold}$) zu berechnen.

Es ergibt sich aus dem Wesen des erfindungsgemäßen Verfahrens, dass der Zahlenwert des Schwellenwertes ($AUC_{threshold}$) bei identischem Versuchsaufbau beziehungsweise konstanten Versuchsbedingungen aufgrund weiterer Patientendaten variieren kann. Mit steigender Kohorte ist von einer weiteren Annäherung des Schwellenwertes ($AUC_{threshold}$) an einen Idealwert auszugehen. Für die Bereitstellung der Testzusammensetzung können unterschiedliche Konzentrationen der Komponenten 5-Fluorouracil, Oxaliplatin und Docetaxel eingesetzt werden. Vorzugsweise kann die Testzusammensetzung eine Komponentenzusammensetzung wie folgt aufweisen: 5-Fluorouracil 10,3 $\mu$M, Oxaliplatin 10,7 $\mu$M und Docetaxel 1,2 nM.

Die Konzentration der mindestens einen Komponente der Testzusammensetzung kann auf Basis von empirisch ermittelten Werten festgelegt werden. Zur empirischen Bestimmung von geeigneten Komponentenkonzentrationen für die Testzusammensetzung werden zunächst Zellen eines ösophago-gastralen Karzinoms, insbesondere eines Magenkarzinoms, von mehreren Patienten entnommen und diese Zellen jeweils separat bei physiologischen Bedingungen *in vitro* kultiviert. Dabei kann die Patientenkultur auf mehrere Vertiefungen einer Zellkulturplatte aufgeteilt werden. Die kultivierten Patientenzellen werden anschließend jeweils mit einer der Komponenten in jeweils vorgegebenen Konzentrationen in Kontakt gebracht und inkubiert. Das heißt, die die Patientenkulturen werden mit den jeweiligen Komponentenkonzentrationen behandelt. Da sich für Leucovorin selbst keine Wirkung auf die Zellen nachweisen lässt, kann Leucovorin als Komponente zur Bestimmung der Komponentenkonzentration ausgenommen werden. Die Behandlung kann in zwei Vorgehensvarianten unterschieden werden. Gemäß einer ersten Variante erfolgt die Behandlung einer Patientenkultur mit den Komponentenkonzentrationen der betreffenden Komponente beginnend mit der niedrigsten Komponentenkonzentration, wobei die Komponentenkonzentration jeweils nach einer vorgegebenen Inkubationszeit schrittweise oder kontinuierlich erhöht wird. Die Zellviabilität wird dabei nach vorgegebenen Inkubationszeiten bestimmt. Gemäß einer zweiten Variante, werden Patientenzellen enthaltene Vertiefungen jeweils mit einer konkreten Konzentration der jeweiligen Komponente behandelt und inkubiert, wobei die Zellviabilitäten nach einer vorgegebenen Inkubationszeit bestimmt werden. Auf diese Weise können mehrere Komponentenkonzentrationen verschiedener Konzentrationen gleichzeitig getestet werden.

Für jede Komponente 5-Fluorouracil, Oxaliplatin und Docetaxel, kann eine patientenspezifische mittlere inhibitorische Konzentration $IC_{50}$ ($IC_{50F}$, $IC_{50O}$, $IC_{50T,}$) ermittelt werden. Die $IC_{50}$-Werte geben an, ab welcher Konzentration der Komponenten eine halbmaximale Wachstumshemmung der Patientenzellen erreicht ist. Anschließend wird aus den Werten der ermittelten patientenspezifischen mittleren inhibitorischen Konzentrationen ($IC_{50F}$, $IC_{50O}$, $IC_{50T}$) für jede Komponente (5-Fluorouracil, Oxaliplatin und Docetaxel, (F, O, T)) ein inhibitorischer Konzentrations-Mittelwert ($mIC_{50F}$, $mIC_{50O}$, $mIC_{50T}$) bestimmt. Der inhibitorische Konzentrations-Mittelwert $mIC_{50}$ ist der Mittelwert aus der Gesamtheit der $IC_{50}$-Werte einer der Komponenten (F, O, T). Es kann vorgesehen werden, dass die $IC_{50}$-Werte der Komponenten, das heißt die Werte $IC_{50F}$, $IC_{50O}$, $IC_{50T}$, zunächst zur Basis 10 logarithmiert werden und aus den logarithmierten Werten ($Log_{10}IC_{50F}$, $Log_{10}IC_{50O}$, $Log_{10}IC_{50T}$) jeweils ein Mittelwert ($mLog_{10}IC_{50F}$, $mLog_{10}IC_{50O}$, $mLog_{10}IC_{50T}$) bestimmt wird, wobei der jeweilige Mittelwert ($mLog_{10}IC_{50}$) sodann exponenziert wird und wobei die Testzusammensetzung mit den Konzentrationen der exponenzierten Konzentrations-Mittelwerte gebildet wird. Auf diese Weise kann einer zu starken Wichtung von hohen $IC_{50}$-Werten gegenüber niedrigen $IC_{50}$-Werten entgegengewirkt werden.

Mit den ermittelten Komponentenkonzentrationen kann sodann die Testzusammensetzung für das erfindungsgemäße Verfahren bereitgestellt werden.

Die Bereitstellung der Testzusammensetzung kann ein eigenes Verfahren darstellen, welches im Sinne der Erfindung als selbstständiger Verfahrensschritt verstanden werden kann. Die auf Basis der ermittelten Komponentenkonzentrationen zusammengestellte Testzusammensetzung weist die Konzentration n auf. Im Folgenden wird der Buchstabe n für die Testzusammensetzung benutzt, welche auf Basis der ermittelten Komponentenkonzentrationen bereitgestellt wurde. Der Buchstabe n steht dabei für eine einfache Konzentration der Testzusammensetzung, wobei 2n beispielsweise für die doppelte Konzentration der Testzusammensetzung steht.

Zur Ermittlung der patientenspezifischen Zellviabilität können mindestens 10 unterschiedliche Konzentrationen der Testzusammensetzung bereitgestellt und eingesetzt werden.

Es hat sich als vorteilhaft erwiesen, dass die Testzusammensetzung zur Ermittlung der patientenspezifischen Zellviabilität in einer 1:2 Verdünnungsreihe von 8n bis 1/64n mit einer konstanten Leucovorinkonzentration von 10 $\mu$M eingesetzt wird. Als Alternative für Leucovorin kann Natriumfolinat mit einer Konzentration von 10 $\mu$M eingesetzt werden.

Mit Hilfe des erfindungsgemäßen Verfahrens kann das *in vivo* Ansprechen von Patienten mit einem ösophago-gastralen Karzinom auf eine Behandlung mit dem FLOT-Chemotherapie-Schema vorausgesagt werden. Dadurch können Patienten, bei denen das erfindungsgemäße Verfahren ein schlechtes Ansprechen voraussagt, einer alternativen Therapie zugeführt werden und damit vor Nebenwirkungen durch das FLOT-Chemotherapie-Schema bewahrt werden.

Weitere Einzelheiten, Merkmale und Vorteile von Ausgestaltungen der Erfindung ergeben sich aus der nachfolgenden Beschreibung von Ausführungsbeispielen mit Bezugnahme auf die zugehörigen Zeichnungen. Es zeigen:

Fig. 1: Diagramme zur näheren Erläuterung des Verfahrensschritts zur Bereitstellung der Testzusammensetzung,

Fig. 2: Diagramme zur näheren Erläuterung des erfindungsgemäßen Vorgehens zur Prädikation eines patientenindividuellen Ansprechens auf das FLOT-Chemotherapie-Schema,

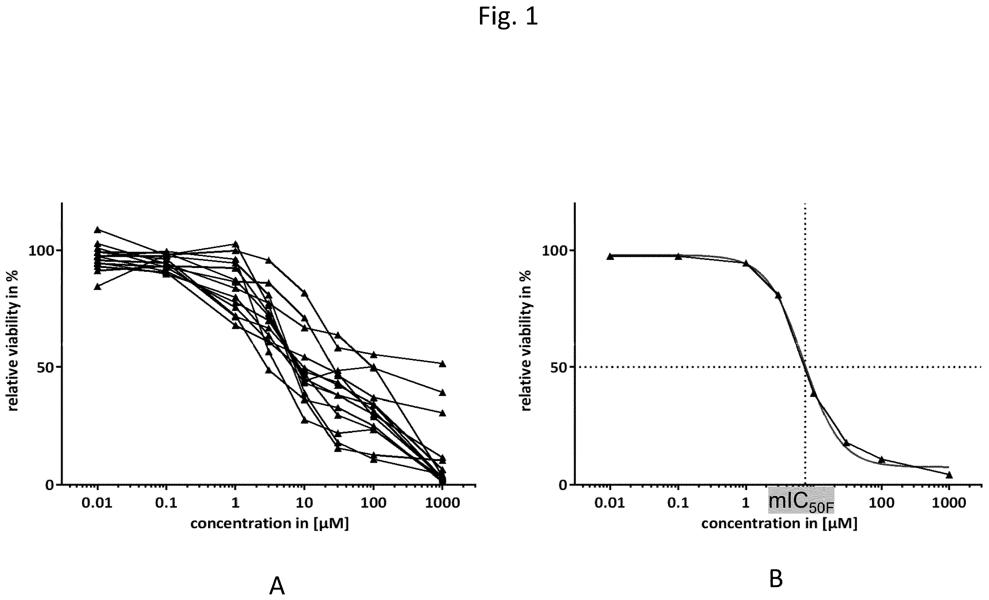

**Figur 1** zeigt Diagramme A und B zur näheren Erläuterung der Bereitstellung der Testzusammensetzung am Beispiel der Komponente 5-Fluorouracil.

Bei der Bereitstellung der Testzusammensetzung wird beispielhaft so vorgegangen, dass zunächst Zellen eines ösophago-gastralen Karzinoms oder eines Magenkarzinoms von mehreren Patienten entnommen und diese Zellen jeweils bei physiologischen Bedingungen *in vitro* kultiviert werden. Im Ausführungsbeispiel wurden Zellen von 13 Patienten eingesetzt. Die kultivierten Patientenzellen werden anschließend mit der Komponente 5-Fluorouracil (5-FU) in mehreren vorgegebenen Konzentrationen behandelt und für eine Dauer von 144 Stunden inkubiert. Das heißt, es werden verschiedene Konzentrationen der Komponente 5-Fluorouracil (5-FU) erstellt, wobei jede Patientenkultur mit einer vorgegebenen Konzentration 5-FU in Kontakt gebracht und inkubiert wird. Nach der Inkubationszeit wird die Zellviabilität jeder Patientenkultur bestimmt. Die Bestimmung der Zellviabilität kann unter Verwendung bekannter Verfahren und Testeinrichtungen erfolgen. Beispielsweise können die Patientenkulturen zur Bestimmung der Zellviabilität für 3 Stunden mit *Presto Blue Cell Reagent* inkubiert und die Fluoreszenz bei 560/590 nm unter Verwendung eines Varioscan Lux der Firma Thermo Fischer Scientific gemessen werden. Anhand der Zellviabilitätswerte wird für jede Komponente eine patientenspezifische Dosis-Wirkungs-Kurve erstellt.

Die erhaltenen patientenspezifischen Dosis-Wirkungs-Kurven der Komponente 5-FU ist in dem Diagramm A gezeigt. Die Ordinatenachse zeigt die relative Zellviabilität in Prozent, wobei die Abszissenachse die Konzentration der Komponente 5-FU in dekadisch logarithmischer Darstellung zeigt. Es handelt sich um 13 Dosis-Wirkungs-Kurven, welche die patientenspezifischen Zellviabilitätswerte von verschiedenen Konzentrationen von 5-FU zeigen.

Das Diagramm B der Figur 1 zeigt eine entsprechend interpolierte Dosis-Wirkungskurve der Komponente 5-FU. Anhand der für 5-FU erstellten interpolierten Kurve wird die mittlere inhibitorische Konzentration $IC50_F$ ermittelt. Die $IC50_F$-Werte der analysierten Zellkulturen von 13 Patienten werden logarithmiert, gemittelt und exponenziert, sodass eine 5-FU-spezifische mittlere halbmaximale inhibitorische Konzentration $mIC50_F$ von 10,3 $\mu$M erhalten wird.

Für die Komponenten Oxaliplatin und Docetaxel konnten gemäß des Ausführungsbeispiels mit ebenfalls jeweils analysierten Zellkulturen von 13 Patienten in gleicher Weise eine Oxaliplatin-spezifische mittlere halbmaximale inhibitorische Konzentration $mIC_{50O}$ von 10,7 $\mu$M sowie sowie eine Docetaxelspezifische mittlere halbmaximale inhibitorische Konzentration $mIC_{50T}$ von 1,2 nM ermittelt werden.

Zur Bestimmung geeigneter Konzentrationen der Komponenten Oxaliplatin und Docetaxel kann gleichermaßen vorgegangen werden.

Anhand der inhibitorischen Konzentrations-Mittelwerte $mIC_{50F}$, $mIC_{50O}$ und $mIC_{50T}$ wird eine Testzusammensetzung bereitgestellt, indem als Komponentenkonzentrationen die ermittelten inhibitorischen Konzentrations-Mittelwerte $mIC_{50F}$, $mIC_{50O}$ und $mIC_{50T}$ eingesetzt werden. Diese Testzusammensetzung wird folgend als Basis genutzt, um das Ansprechen der Patientenkulturen zu testen.

Hierfür wird die Testzusammensetzung in 2-fach Schritten konzentriert und in 1:2 Schritten verdünnt, so dass 10 verschiedene Behandlungskonzentrationen von 8n bis 1/64n entstehen. Da Leucovorin in Einzeltestungen keinen Effekt auf die Zellviabilität der Patientenkulturen zeigt, wird Leucovorin als Additiv zur Unterstützung der Wirkung von 5-FU eingesetzt. Die Konzentration von Leucovorin beträgt in allen Verdünnungsstufen der Testzusammensetzung konstant 10 $\mu$M. Die Zellviabilitätsmessungen der 10 Behandlungskonzentrationen ergeben die Viabilitätskurve.

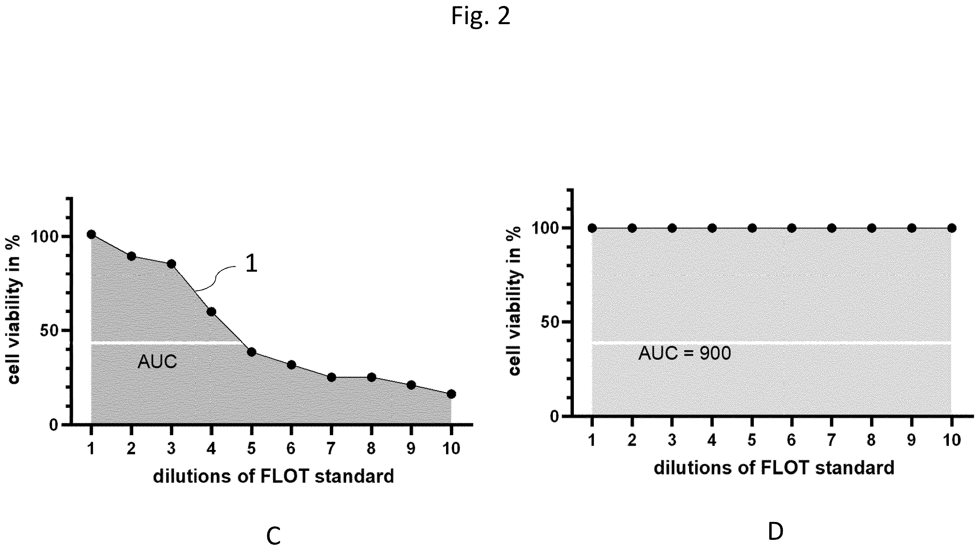

Die **Figur 2** zeigt Diagramme zur näheren Erläuterung des erfindungsgemäßen Verfahrens zur Prädikation eines patientenindividuellen Ansprechens auf das klinische FLOT-Chemotherapie-Schema.

Bei dem Verfahren zur Prädikation eines patientenindividuellen Ansprechens auf ein FLOT-Chemotherapie-Schema bei ösophago-gastralen Karzinomen, werden zunächst Zellen eines Karzinoms eines Patienten entnommen und bei physiologischen Bedingungen *in vitro* kultiviert. Die Patientenkultur wird in einer Zellkulturplatte mit mindestens zehn Vertiefungen ausgebracht. Am Folgetag wird jede Vertiefung mit einer vorgegebenen Behandlungskonzentration der bereitgestellten Testzusammensetzung behandelt und für 72 Stunden inkubiert. Im Anschluss an die erste Inkubationszeit wird dieser Vorgang wiederholt. Nach einer Gesamtinkubationszeit von 144 Stunden wird eine Zellviabilität der Patientenzellen der einzelnen Vertiefungen ermittelt. Die nachfolgende Tabelle zeigt beispielhaft die ermittelten Zellviabilitätswerte von mit vorgegebenen Konzentrationen der verwendeten Testzusammensetzung inkubierten Zellen eines Patienten X. Die Testzusammensetzung wurde in einer 1:2 Verdünnungsreihe von 8n bis 1/64n gemäß der nachfolgend gezeigten Tabelle 1 eingesetzt.

Tabelle 1

| Lösung | 1 | 2 | 3 | 4 | 5 | 6 | 7 | 8 | 9 | 10 |
|---|---|---|---|---|---|---|---|---|---|---|
| Konzentration der Testzusammensetzung | 1/64n | 1/32n | 1/16n | 1/8n | 1/4n | 1/2n | n | 2n | 4n | 8n |
| Zellviabilitätswert Patient X | 101,3 | 89,6 | 85,5 | 59,9 | 38,8 | 32,0 | 25,4 | 25,4 | 21,2 | 16,4 |

Die ermittelten Zellviabilitätswerte werden in einem Diagramm abgebildet, wobei die Ordinatenachse die Zellviabilität in Prozent zeigt und die Abszissenachse zur Abbildung der Nummerierung der Konzentrationen der Testzusammensetzung in der Konzentration 1/64n bis 8n dient. Ein gemäß der vorstehenden Tabelle erstelltes Diagramm C ist der Figur 2 zu entnehmen. Die aufgrund der Tabellenwerte durch Verbinden der Punkte erhaltene Funktion 1, welche auch als Kurve bezeichnet werden kann, wird zur Bestimmung eines patientenspezifischen Wertes $AUC_{rel}$ eingesetzt. Zur Berechnung des patientenspezifischen Wertes $AUC_{rel}$ wird zunächst das Integral AUC der erhaltenen Funktion 1 ermittelt. Aus der generierten Funktion 1 ergibt sich ein AUC-Wert von 436,6. Anschließend wird der Quotient aus dem AUC-Wert und der maximal möglichen Fläche $AUC_{max}$, welche der konstanten (unveränderten) maximalen Zellviabilität entspricht, gebildet. Dieser Quotient entspricht dem patientenspezifischen Wert $AUC_{rel}$, welcher eine Diskriminierung von auf die Testzusammensetzung ansprechende Patientenzellen ermöglicht, indem der patientenspezifische Wert $AUC_{rel}$ mit einem festgelegten Schwellenwert $AUC_{threshold}$, welcher im Ausführungsbeispiel 0,559 beträgt, verglichen wird. Der Schwellenwert $AUC_{threshold}$ 0,559 wurde in einer klinischen Studie ermittelt, in welcher das *in vivo* Ansprechen im Patienten anhand der pathologischen Auswertung der prozentual viablen Tumorzellen nach Chemotherapie mit dem FLOT-Chemotherapie-Schema mit dem *in vitro* Ansprechen der Tumorzellkulturen verglichen wurde. Bei diesem Ausführungsbeispiel zeigt ein $AUC_{rel}$ kleiner als 0,559 ein gutes Ansprechen auf eine Behandlung mit dem FLOT-Chemotherapie-Schema und eine $AUC_{rel}$ größer als 0,559 zeigt ein schlechtes Ansprechen auf eine Behandlung mit dem FLOT-Chemotherapie-Schema. Ein Vergleich mit einem vorgegebenen Schwellenwert $AUC_{threshold}$ ermöglicht somit eine Prädikation eines patientenindividuellen Ansprechens auf die vorgegebene Behandlung mit dem FLOT-Chemotherapie-Schema.

**Bezugszeichenliste**

1 Funktion

**Patentansprüche**

1. Verfahren zur Prädikation eines patientenindividuellen Ansprechens auf ein FLOT-Chemotherapie-Schema bei ösophago-gastralen Karzinomen, bei welchem Zellen eines ösophago-gastralen Karzinoms, insbesondere eines Magenkarzinoms, eines Patienten bei physiologischen Bedingungen *in vitro* kultiviert werden, die kultivierten Patientenzellen mit mindestens einer vorgegebenen Konzentration einer Testzusammensetzung enthaltend mindestens eine Komponente ausgewählt aus einer Gruppe von Komponenten aufweisend 5-Fluorouracil (5-FU), Oxaliplatin und Docetaxel, inkubiert werden, und nach einer vorgegebenen Inkubationsdauer eine Zellviabilität der Patientenzellen ermittelt wird, anhand welcher ein patientenindividuelles Ansprechen der Patientenzellen auf das FLOT-Chemotherapie-Schema abgeleitet wird, wobei anhand der ermittelten Zellviabilitätswerte eines Patienten eine Dosis-Wirkungs-Kurve erstellt wird, wobei der patientenspezifische Wert ($AUC_{rel}$) aus dem Quotient des Integrals der Dosis-Wirkungs-Kurve und dem Integral der Funktion der Zellviabilitätswertmaxima ($AUC_{max}$) ermittelt wird, und wobei ein patientenspezifischer Wert ($AUC_{rel}$) kleiner als der vorgegebene Schwellenwert ($AUC_{threshold}$) als ein gutes Ansprechen und ein patientenspezifischer Wert größer als der Schwellenwert ($AUC_{threshold}$) als ein schlechtes Ansprechen auf die FLOT-Standardzusammensetzung (n) bewertet werden, wobei der Schwellenwert ($AUC_{threshold}$) auf Basis einer *in vitro* Dosis-Wirkung bestimmt wird.

2. Verfahren nach Anspruch 1, **dadurch gekennzeichnet, dass** die mindestens eine Komponente der Testzusammensetzung eine vorgegebene Komponentenkonzentration aufweist.

3. Verfahren nach einem der vorhergehenden Ansprüche, **dadurch gekennzeichnet, dass** die Patientenkultur in Vertiefungen einer Zellkulturplatte geteilt wird und die einzelnen Vertiefungen mit den darin enthaltenen Patientenzellen mit unterschiedlichen Konzentrationen der Testzusammensetzung behandelt werden.

4. Verfahren nach einem der vorhergehenden Ansprüche, **dadurch gekennzeichnet, dass** ein auf einem Computer ausgeführtes Programm angewendet wird, um den Schwellenwert ($AUC_{threshold}$) anhand von *in vitro* bestimmten Dosis-Wirkungs-Werten zu berechnen.

5. Verfahren nach einem der vorhergehenden Ansprüche, **dadurch gekennzeichnet, dass** die bereitgestellte Testzusammensetzung folgende Komponentenkonzentrationen aufweist:

   - 5-Fluorouracil (F) 10,3 $\mu$M
   - Oxaliplatin (O) 10,7 $\mu$M
   - Docetaxel (T) 1,2 nM.

6. Verfahren nach einem der vorhergehenden Ansprüche, **dadurch gekennzeichnet, dass** zur Bereitstellung der Testzusammensetzung zunächst Zellen eines ösophago-gastralen Karzinoms, insbesondere eines Magenkarzinoms von mehreren Patienten jeweils bei physiologischen Bedingungen *in vitro* kultiviert werden, die kultivierten Patientenzellen anschließend jeweils mit einer der Komponenten 5-Fluorouracil, Oxaliplatin und/oder Docetaxel in mehreren vorgegebenen Konzentrationen behandelt werden und für jede Komponente 5-Fluorouracil, Oxaliplatin und/oder Docetaxel eine patientenspezifische mittlere inhibitorische Konzentration ($IC_{50F}$, $IC_{50O}$, $IC_{50T}$) ermittelt wird, und aus allen Werten der ermittelten patientenspezifischen mittleren inhibitorischen Konzentrationen für jede Komponente 5-Fluorouracil, Oxaliplatin und/oder Docetaxel ein inhibitorischer Konzentrations-Mittelwert ($mIC_{50F}$, $mIC_{50O}$, $mIC_{50T}$) errechnet wird, wobei die Testzusammensetzung mit den Konzentrationen der inhibitorischen Konzentrations-Mittelwerte ($mIC_{50F}$, $mIC_{50O}$, $mIC_{50T}$) gebildet wird.

7. Verfahren nach dem vorhergehenden Anspruch, **dadurch gekennzeichnet, dass** die $IC_{50}$-Werte der Komponenten zunächst zur Basis 10 logarithmiert werden und aus den logarithmierten Werten ($Log_{10}IC_{50F}$, $Log_{10}IC_{50O}$, $Log_{10}IC_{50T}$) jeweils ein Mittelwert ($mLog_{10}IC_{50F}$, $mLog_{10}IC_{50O}$, $mLog_{10}IC_{50T}$) bestimmt wird, wobei der jeweilige Mittelwert ($mLog_{10}IC_{50F}$, $mLog_{10}IC_{50}$, $mLog_{10}IC_{50T}$) sodann exponenziert wird, wobei die Testzusammensetzung mit den Konzentrationen der exponenzierten Konzentrations-Mittelwerte gebildet wird.

8. Verfahren nach einem der vorhergehenden Ansprüche, **dadurch gekennzeichnet, dass** zur Ermittlung der patientenspezifischen Zellviabilität mindestens 10 unterschiedliche Konzentrationen der Testzusammensetzung eingesetzt werden.

9. Verfahren nach einem der vorhergehenden Ansprüche, **dadurch gekennzeichnet, dass** die Testzusammensetzung zur Ermittlung der patientenspezifischen Zellviabilität in einer 1:2 Verdünnungsreihe von 8n bis 1/64n mit einer konstanten Leucovorin-Konzentration von 10 $\mu$M eingesetzt wird.

10. Verfahren nach einem der vorhergehenden Ansprüche, **dadurch gekennzeichnet, dass** die Patientenzellen für eine Zeitdauer von mindestens 144 Stunden inkubiert werden, bevor die Zellviabilität bestimmt wird.

**Claims**

1. A method for predicting a patient-specific response to a FLOT chemotherapy regimen for oesophagogastric carcinomas, in which cells of an oesophagogastric carcinoma, in particular of a gastric carcinoma, of a patient are cultivated *in vitro* under physiological conditions, the cultivated patient cells are incubated with at least one specified concentration of a test composition containing at least one component selected from a group of components comprising 5-fluorouracil (5-FU), oxaliplatin and docetaxel, and, after a specified incubation period, the cell viability of the patient cells is determined, from which a patient-specific response of the patient cells to the FLOT chemotherapy regimen is derived, wherein a dose-response curve is created using the determined cell viability values of a patient, wherein the patient-specific value ($AUC_{rel}$) is determined from the quotient of the integral of the dose-response curve and the integral of the function of the cell viability value maxima ($AUC_{max}$), and wherein a patient-specific value ($AUC_{rel}$) of less than the specified threshold value ($AUC_{threshold}$) is assessed as a good response, and a patient-specific value of more than the threshold value ($AUC_{threshold}$) is assessed as a poor response to the FLOT standard composition (n), wherein the threshold value ($AUC_{threshold}$) is determined on the basis of an *in-vitro* dose-response.

2. The method according to Claim 1, **characterised in that** the at least one component of the test composition has a specified component concentration.

3. The method according to one of the preceding claims, **characterised in that** the patient culture is divided into wells of a cell culture plate, and the individual wells containing the patient cells are treated with different concentrations of the test composition.

4. The method according to one of the preceding claims, **characterised in that** a program executed on a computer is used to calculate the threshold value ($AUC_{threshold}$) using dose-response values determined *in vitro*.

5. The method according to one of the preceding claims, **characterised in that** the prepared test composition has the following component concentrations:

   - 5-fluorouracil (F) 10.3 $\mu$M
   - oxaliplatin (O) 10.7 $\mu$M
   - docetaxel (T) 1.2 nM.

6. The method according to one of the preceding claims, **characterised in that,** to prepare the test composition, cells of an oesophagogastric carcinoma, in particular of a gastric carcinoma, of multiple patients are first cultivated *in vitro* under physiological conditions, the cultivated patient cells are then treated with one of the components 5-fluorouracil, oxaliplatin and/or docetaxel in multiple specified concentrations, and a patient-specific mean inhibitory concentration ($IC_{50F}$, $IC_{50O}$, $IC_{50T}$) is determined for each component 5-fluorouracil, oxaliplatin and/or docetaxel, and an inhibitory concentration mean value ($mIC_{50F}$, $mIC_{50O}$, $mIC_{50T}$) is calculated from all the values of the determined patient-specific mean inhibitory concentrations for each component 5-fluorouracil, oxaliplatin and/or docetaxel, wherein the test composition is formed with the concentrations of the inhibitory concentration mean values ($mIC_{50F}$, $mIC_{50O}$, $mIC_{50T}$).

7. The method according to the preceding claim, **characterised in that** the $IC_{50}$ values of the components are first logarithmised to base 10, and a mean value ($mLog_{10}IC_{50F}$, $mLog_{10}IC_{50O}$, $mLog_{10}IC_{50T}$) is determined from each of the logarithmised values ($Log_{10}IC_{50F}$, $Log_{10}IC_{50O}$, $Log_{10}IC_{50T}$), wherein each mean value ($mLog_{10}IC_{50F}$, $mLog_{10}IC_{50O}$, $mLog_{10}IC_{50T}$) is then exponentiated, wherein the test composition is formed with the concentrations of the exponentiated concentration mean values.

8. The method according to one of the preceding claims, **characterised in that** at least 10 different concentrations of the test composition are used to determine the patient-specific cell viability.

9. The method according to one of the preceding claims, **characterised in that** the test composition is used in a 1:2 dilution series of 8n to 1/64n with a constant leucovorin concentration of 10 $\mu$M to determine the patient-specific cell viability.

10. The method according to one of the preceding claims, **characterised in that** the patient cells are incubated for a period of at least 144 hours before the cell viability is determined.


## Revendications

1. Procédé de prédiction de la réponse individuelle d'un patient à un schéma de chimiothérapie FLOT dans le cas de carcinomes œsophago-gastriques, dans lequel des cellules d'un carcinome œsophago-gastrique d'un patient, en particulier d'un carcinome gastrique, sont cultivées in vitro dans des conditions physiologiques, les cellules du patient ainsi cultivées sont ensuite incubées avec au moins une concentration prédéfinie d'une composition d'essai contenant au moins un composant sélectionné parmi un groupe de composants présentant le 5-fluorouracile (5-FU), l'oxaliplatine et le docétaxel, puis, après une durée d'incubation prédéfinie, on procède à la détermination d'une viabilité des cellules du patient, laquelle sert alors à déduire une réponse individuelle des cellules du patient au schéma de chimiothérapie FLOT, une courbe dose-effet étant établie à partir des valeurs de viabilité cellulaire déterminées chez un patient, la valeur spécifique au patient (AUCrel) étant déterminée à partir du quotient de l'intégrale de la courbe dose-effet et de l'intégrale de la fonction des valeurs maximales de viabilité cellulaire (AUCmax), et une valeur spécifique au patient (AUCrel) inférieure à la valeur seuil prédéfinie (AUCthreshold) étant interprétée comme indiquant une bonne réponse à la composition standard FLOT (n), et une valeur spécifique au patient supérieure à la valeur seuil (AUCthreshold) étant interprétée comme indiquant une mauvaise réponse à ladite composition standard FLOT (n), la valeur seuil (AUCthreshold) étant déterminée sur la base d'une relation dose-effet obtenue in vitro.

**2.** Procédé selon la revendication 1, **caractérisé en ce que** ledit au moins un composant de la composition d'essai présente une concentration prédéterminée.

**3.** Procédé selon l'une quelconque des revendications précédentes, **caractérisé en ce que** la culture des cellules du patient est répartie dans des puits d'une plaque de culture cellulaire et que les puits individuels contenant les cellules du patient sont traités avec la composition d'essai à différentes concentrations.

**4.** Procédé selon l'une quelconque des revendications précédentes, **caractérisé en ce qu'**un programme exécuté sur un ordinateur est utilisé pour calculer la valeur seuil ($AUC_{threshold}$) à partir de valeurs dose-effet déterminées *in vitro.*

**5.** Procédé selon l'une quelconque des revendications précédentes, **caractérisé en ce que** la composition d'essai fournie présente les concentrations de composants suivantes :

- 5-fluorouracile (F) 10,3 $\mu$M
- oxaliplatine (0) 10,7 $\mu$M
- docétaxel (T) 1,2 nM.

**6.** Procédé selon l'une quelconque des revendications précédentes, **caractérisé en ce que** la préparation de la composition d'essai est obtenue tout d'abord en cultivant *in vitro,* dans des conditions physiologiques, des cellules d'un carcinome œsophago-gastrique, en particulier d'un carcinome gastrique provenant de plusieurs patients, puis en traitant les cellules des patients ainsi cultivées avec chaque composant, le 5-fluorouracile, l'oxaliplatine et/ou le docétaxel, à plusieurs concentrations prédéfinies, et en déterminant pour chaque composant, le 5-fluorouracile, l'oxaliplatine et/ou le docétaxel, une concentration inhibitrice médiane spécifique au patient ($IC_{50F}$, $IC_{50O}$, $IC_{50T}$), et en calculant pour chaque composant, le 5-fluorouracile, l'oxaliplatine et/ou le docétaxel, une valeur moyenne de concentration inhibitrice médiane ($mIC_{50F}$, $mIC_{50O}$, $mIC_{50T}$) à partir de toutes les valeurs des concentrations inhibitrices médianes déterminées spécifiques aux patients, la composition d'essai étant formée en utilisant les concentrations obtenues à partir des valeurs moyennes des concentrations inhibitrices médianes ($mIC_{50F}$, $mIC_{50O}$, $mIC_{50T}$.

**7.** Procédé selon la revendication précédente, **caractérisé en ce que** les valeurs $IC_{50}$ des composants sont tout d'abord converties en logarithmes à base 10 et une valeur moyenne ($mLog_{10}IC_{50F}$, $mLog_{10}IC_{50O}$, $mLog_{10}IC_{50T}$) est alors respectivement déterminée à partir des valeurs logarithmiques ($Log_{10}IC_{50F}$, $Log_{10}IC_{50O}$, $Log_{10}IC_{50T}$), la valeur moyenne respective ($mLog_{10}IC_{50F}$, $mLog_{10}IC_{50O}$, $mLog_{10}IC_{50T}$) étant ensuite mise en puissance, la composition d'essai étant formée en utilisant les concentrations obtenues à partir des valeurs moyennes des concentrations mises en puissance.

**8.** Procédé selon l'une quelconque des revendications précédentes, **caractérisé en ce que,** pour déterminer la viabilité cellulaire spécifique au patient, la composition d'essai est utilisée à au moins 10 concentrations différentes.

**9.** Procédé selon l'une quelconque des revendications précédentes, **caractérisé en ce que,** pour déterminer la viabilité cellulaire spécifique au patient, la composition d'essai est utilisée dans une série de dilutions 1:2 allant de 8n à 1/64n et présentant une concentration constante de leucovorine de 10 $\mu$M.

**10.** Procédé selon l'une quelconque des revendications précédentes, **caractérisé en ce que** les cellules du patient sont incubées pendant une durée d'au moins 144 heures avant que la viabilité cellulaire ne soit déterminée.

Fig. 1

A

B

EP 4 382 907 B1

Fig. 2

C

D

EP 4 382 907 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3657167 A